(19) Europäisches Patentamt
European Patent Office
Office européen des brevets

(11) Publication number: **0 112 481**
**A1**

(12) **EUROPEAN PATENT APPLICATION**

(21) Application number: **83111375.8**

(22) Date of filing: **14.11.83**

(51) Int. Cl.³: **C 07 D 471/04**, A 61 K 31/435
// (C07D471/04, 221/00, 205/00)

(30) Priority: **12.11.82 JP 198719/82**
**23.04.83 JP 72034/83**

(43) Date of publication of application: **04.07.84**
**Bulletin 84/27**

(84) Designated Contracting States: **DE FR GB**

(71) Applicant: **KYOWA HAKKO KOGYO CO., LTD,**
**Ohtemachi Bldg. Ohtemachi 1-chome Chiyoda-ku, Tokyo (JP)**

(72) Inventor: **Hirata, Tadashi, 1566-315, Nara-cho Midori-ku, Yokohama-shi Kanagawa-ken (JP)**
Inventor: **Sato, Akira, 1880-30, Kiso-machi, Machida-shi Tokyo (JP)**

(74) Representative: **Casalonga, Axel et al, BUREAU D.A. CASALONGA OFFICE JOSSE & PETIT**
**Baaderstrasse 12-14, D-8000 München 5 (DE)**

(54) **Beta-lactam compound.**

(57) β-Lactam compounds represented by the general formula:

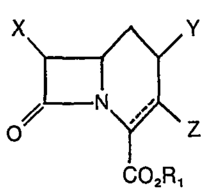

[wherein X is phthalimido, azido, phenylacetamido, phenoxyacetamido, 2-thienylacetamido, or

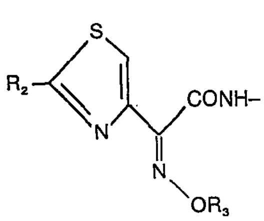

(wherein $R_2$ is amino or protected amino, $R_3$ is hydrogen or methyl); Y is hydrogen, methyl or hydroxyl; Z is $-CHR_4NO_2$ (wherein $R_4$ is hydrogen or straight or branched alkyl of $C_1-C_4$) or formyl; $R_1$ is straight or branched alkyl of $C_1-C_5$ (which can be substituted by halogen), substituted silyl, or benzyl, benzhydryl or trityl (whose phenyl nuclei can be substituted by methyl, methoxy or nitro); ----- represents single bond when Z is $-CHR_4NO_2$ and represents single or double bond when Z is formyl; and in spite of the above definition, when X is

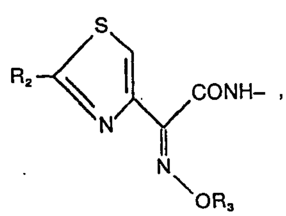

Z is $-CHR_4NO_2$] are useful intermediates which can be converted to compounds having an antibacterial activity.

EP 0 112 481 A1

# TITLE OF INVENTION

## β-LACTAM COMPOUND

## Background of the Invention

The present invention relates to a novel β-lactam compound, more specifically to a carbacephem or carbacepham compound wherein hydrogen atom at the 3-position is substituted by an unsubstituted or substituted nitromethyl group or a formyl group.

As to carbacephem compounds with a substituent at the 3-position, those with a halogen atom at the 3-position (GB 2041923A), those with methyl, hydroxymethyl, etc. (US Patent No. 4,226,866), etc. have heretofore been known. The Compounds of the present invention are novel compounds not yet disclosed in any literature, and are useful intermediates which can be converted to compounds having an antibacterial activity, as will be described later.

## Detailed Description of the Invention

This invention relates to a novel β-lactam compound represented by the general formula [I]:

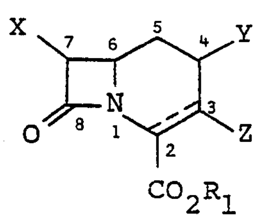

[I]

[wherein X is phthalimido, azido, phenylacetamido, phenoxyacetamido, 2-thienylacetamido, or

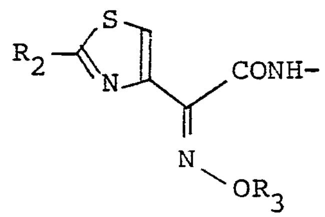

(wherein $R_2$ is amino or protected amino, $R_3$ is hydrogen or methyl); Y is hydrogen, methyl or hydroxyl; Z is $-CHR_4NO_2$ (wherein $R_4$ is hydrogen or straight or branched alkyl of $C_1 - C_4$) or formyl; $R_1$ is straight or branched alkyl of $C_1 - C_5$ (which can be substituted by halogen), substituted silyl, or benzyl, benzhydryl or trityl (whose phenyl nuclei can be substituted by methyl, methoxy or nitro); ----- represents single bond when Z is $-CHR_4NO_2$ and represents single or double bond when Z is formyl; and in spite of the above definition, when X is 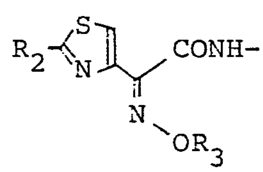 , Z is $-CHR_4NO_2$] (hereinafter referred to as "Compound [I]" or merely [I], and compounds of other formulae shall likewise be referred to).

The protecting group of "protected amino group" for $R_2$ in the general formula [I] can be exemplified by trityl, t-butyloxycarbonyl, 2,2,2-trichloroethyloxycarbonyl, chloroacetyl, formyl, etc.

The "straight or branched alkyl group of $C_1 - C_4$" for $R_4$ can be exemplified by methyl, ethyl, propyl, i-propyl, butyl, i-butyl, sec-butyl, tert-butyl, etc.

The "straight or branched alkyl group of $C_1 - C_5$" for $R_1$ can be exemplified by methyl, ethyl, tert-butyl, etc.

The group substituted with halogen in the $R_1$ group can be exemplified by chloromethyl, chloroethyl, 2,2,2-trichloroethyl, 2,2,2-trifluoroethyl, etc., and the halogen can be exemplified by chlorine, fluorine, and

BAD ORIGINAL

bromine.

The substituted silyl can be exemplified by trimethylsilyl, t-butyldimethylsilyl, etc.

A Compound [I] wherein Z is $-CHR_4NO_2$ and - - - - represents single bond, namely a compound represented by the general formula [I-1]:

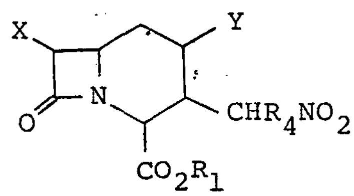

[ I-1 ]

(wherein X, Y, $R_1$ and $R_4$ have the same meanings as defined above) can be obtained by reacting a known compound represented by the general formula [II]:

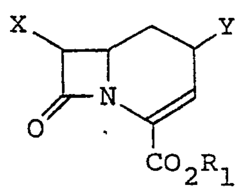

[ II ]

(wherein X, Y and $R_1$ have the same meanings as defined above) and disclosed in GB 2017102A, etc. with a compound of general formula [III]:

$$H_2CR_4NO_2 \qquad [\ III\ ]$$

(wherein $R_4$ has the same meaning as defined above) in the presence of a base.

Michael addition-type reaction to form a direct carbon-carbon bond at the 3-position of 3H-β-lactam is a novel reaction not disclosed in any literature with respect to not only carbacephems but also 3H-cephalosporins.

The Compound [III] can be exemplified by nitromethane, nitroethane, 1-nitropropane, etc. The base can be exemplified by triethylamine, 1,5-diazabicyclo[4.3.0]-

nonene-5, 1,8-diazabicyclo [5.4.0] undecene-7, piperazine, pyrrolidine, tetramethylguanidine, pyridine, etc.

The amount of the base ranges from a catalytic amount to 2 equivalents to the Compound [II] depending upon reaction conditions and generally, a preferred amount is 0.1 - 1.0 equivalent. A solvent for use in the reaction is chloroform, methylene chloride, 1,2-dimethoxyethane, tetrahydrofuran, dioxane, diethyl ether, toluene, ethyl acetate, dimethylsulfoxide, N,N-dimethylformamide or Compound [III] as the reaction reagent, or a mixture thereof.

The reaction temperature is 0 - 100°C, preferably 0 - 50°C, and the reaction time is 30 minutes to 7 days, being dependent upon reaction conditions.

Isolation and purification of Compound [I-1] from the reaction mixture can be carried out according to the ordinary procedure used in the synthesis of penicillins or cephalosporins, for example, by extraction, column chromatography, recrystallization, etc.

A Compound [I-1] can be converted to well known cephalosporin analogs [IV], [V] and [VI] by the following novel processes I and II found by the present inventors and given below. These processes will be illustrated by Reference Examples:

(Process I)

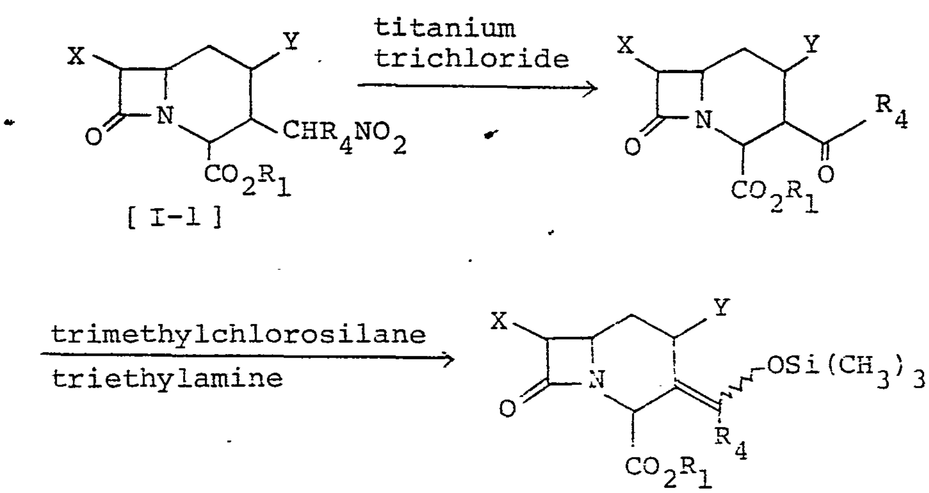


[ I-1 ]

BAD ORIGINAL

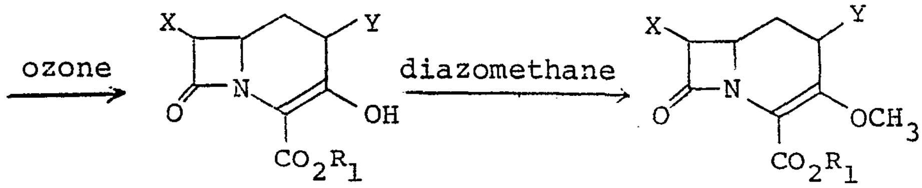


[ IV ] [ V ]

(wherein X, Y, $R_1$ and $R_4$ have the same meanings as defined above).

Carbacephem compounds having hydroxyl or methoxy at the 3-position such as Compound [IV] and [V] are disclosed in Chem. Pharm. Bull 28 (5) 1563 (1980).

(Process II)

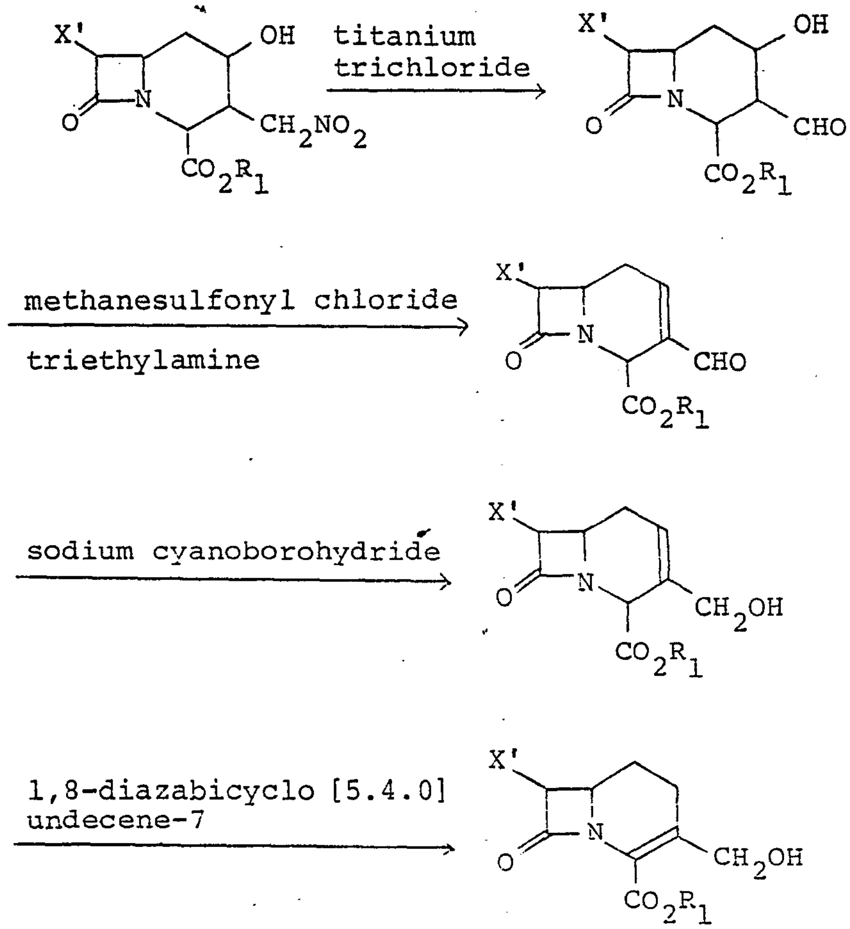


[ VI ]

(wherein X' has the same meaning as X except the azido group).

Carbacephem compounds having 3-hydroxymethyl such as compound (VI) are also disclosed in the Chem. Pharm. Bull.

A compound (I) wherein Z is formyl, ---- represents single or double bond and 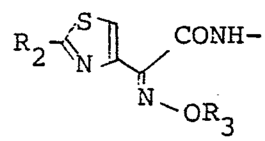 is excluded from the definition of X, namely a compound represented by the general formula [I-2]:

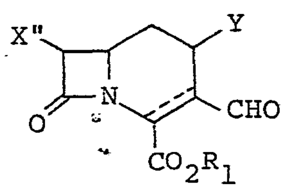

[ I-2 ]

(wherein Y and $R_1$ have the same meanings as defined above, and X" has the same meaning as X except 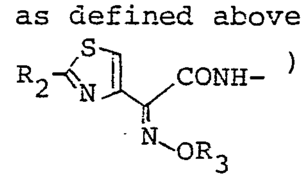 )

can be synthesized from a Compound [I-1] wherein X is X", namely a compound represented by the general formula [I-3]:

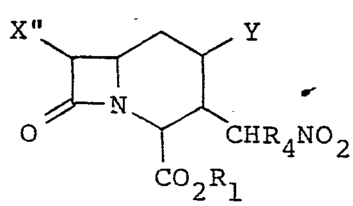

[ I-3 ]

(wherein X", Y, $R_1$ and $R_4$ have the same meanings as defined above) according to the following process:

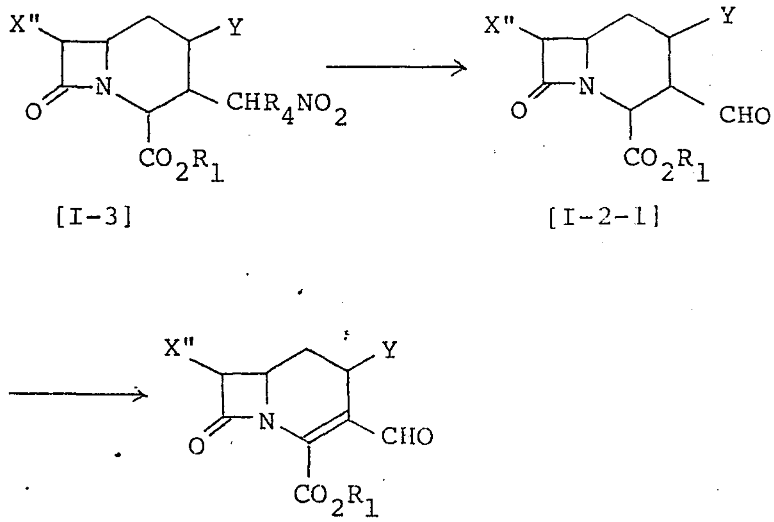

[I-3] [I-2-1] [I-2-2]

(wherein X", Y, $R_1$ and $R_4$ have the same meanings as defined above).

Compound [I-2] consists of Compound [I-2-1] and Compound [I-2-2].

Conversion of nitromethyl group to aldehyde group in step [I-3] → [I-2-1] is preferably carried out by Nef reaction (dilute acid treatment), reaction by $MoO_5$ · pyridine · hexamethylphosphoramide after base treatment, ozone oxidation after base treatment, reaction by potassium permanganate after base treatment, reaction by titanium trichloride, etc. The last process is particularly preferable because of mild reaction, and the reference reaction process is disclosed in J. Org. Chem. 38 #26,4367 (1973). Particularly preferably, the nitro Compound [I-3] is subjected to reaction in a mixed system of an aqueous solution of titanium trichloride, and an organic solvent such as methanol, ethanol, 1,2-dimethoxyethane, tetrahydrofuran, dioxane, dimethylformamide, dimethylsulfoxide, etc. at 0 - 30°C. Yield can be improved by carrying out the reaction under a weakly acidic condition by adding ammonium acetate, etc. to the reaction system. In the reaction, 1 - 10 equivalents of titanium trichloride is used on the basis of the substrate.

Introduction of a double bond in the step [I-2-1] → [I-2-2] is carried out by treating [I-2-1] with an appropriate halide to introduce a halogen to the α-position of the aldehyde, i.e. the 3-position and then eliminating hydrogen halide by an appropriate base. In the halogenation of the 3-position a reagent to be usually used for halogenation of the α-position of an aldehyde can be used, and is preferably exemplified by bromine, chlorine, N-bromosuccinimide, N-chlorosuccinimide, N-bromoacetamide, sulfuryl chloride, cupric bromide, cupric chloride, Iodobenzene dichloride, pyrrolidone hydrotribromide, 5,5-dibromo-2,2-dimethyl-4,6-dioxo-1,3-dioxane, etc. Particularly, the last two halogenating agents are preferable.

Any solvent may be used in the reaction so long as it is inert and does not take part in the reaction, and can be exemplified by tetrahydrofuran, dioxane, 1,2-dimethoxyethane, diethyl ether, methylene chloride, chloroform, toluene, acetonitrile, etc. Reaction temperature is -20 to 80°C depending upon the halogenating agent used, and preferred temperature is generally 0 - 60°C. If necessary, an appropriate acid or base can be added to the reaction system to proceed with the halogenation reaction smoothly. The reaction time varies depending upon the halogenating agent, reaction temperature, solvent, etc. used, and usually is 10 minutes to 2 hours. The halide as formed is treated with an appropriate base without the necessity of isolation to obtain the desired Compound [I-2-2]. The base for use in the treatment is exemplified by triethylamine, diethylamine, 1,5-diazabicyclo [4.3.0] nonene-5, 1,8-diazabicyclo[5.4.0]undecene-7, piperazine, pyrrolidine, pyridine, N,N-dimethylaniline, sodium hydrogen carbonate, sodium carbonate, potassium carbonate, calcium carbonate, etc. In the reaction, 1 to 5, preferably 2 to 3 equivalents of the base is used on the basis of the starting Compound [I-2-1]. The reaction temperature is -10 to 50°C, and the reaction is preferably carried out under ice cooling. The reaction time is 30 minutes to 6

hours preferably 1 to 3 hours.

The thus obtained Compound [I-2-2] is a useful intermediate which can be converted to a compound having an antibacterial activity, and an example illustrating how a vinylcarbacephalosporin compound is derived from Compound [I-2-2] is shown below, and its embodiment will be shown in Reference Example.

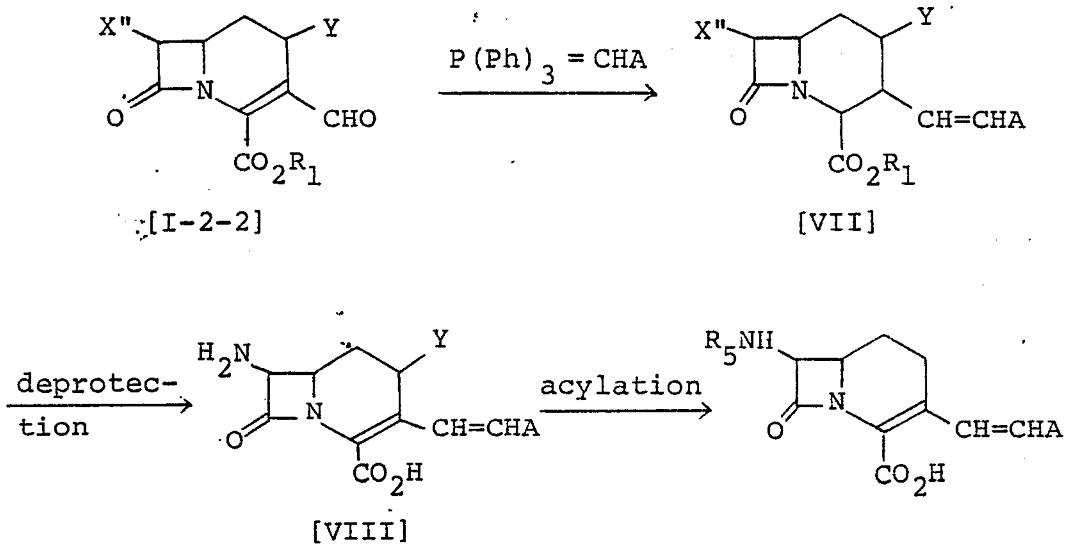

[I-2-2] [VII] [VIII]

In the foregoing process formulae, A is exemplified by ethoxycarbonyl, cyano, 1-methyl-5-tetrazolyl, etc., and $R_5$ represents various acyl groups usually used in the field of penicillin and cephalosporin chemistry.

In Compound [VII] of the above process and Compounds [IV], [V] and [VI], the ester group represented by $CO_2R_1$ can be converted to a carboxyl group without impairing the substituent and functional group in the molecule according to the procedure frequently used in the penicillin and cephalosporin chemistry by selecting appropriate conditions and reagent.

In Compound [VII] of the above process and Compounds [IV], [V] and [VI], when the functional group at the 7-position is an azido group, the azido group can be converted to an amino group preferably by catalytic reduction, or according to a procedure using hydrogen sulfide

in the presence of a base such as triethylamine, pyridine, etc. When the functional group at the 7-position is a phthalimido group, the phthalimido group can be converted to an amino group according to a procedure using a hydrazine compound disclosed in Japanese Published Unexamined Patent Application No. 91991/1982. By introducing various acyl groups usually used in the chemistry of penicillin or cephalosporin into the amino group of Compound [VIII], useful cephalosporin analogs having an antibacterial effect can be obtained.

Embodiments of the present invention will be given in detail below, referring to Examples and Reference Examples.

Example 1

Tertiary butyl ester of (±)-6,7-cis-7-phthalimido-1-azabicyclo [4.2.0] oct-3-nitromethyl-8-oxo-2-carboxylic acid:

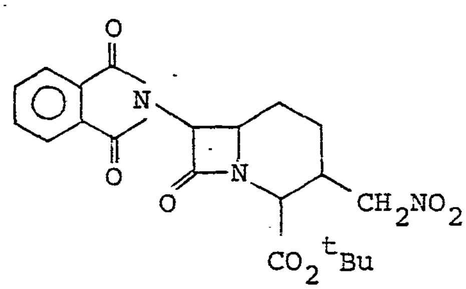

At first, 6 g (16.3 m moles) of tertiary butyl ester of (±)-cis-7-phthalimido-1-azabicyclo [4.2.0] oct-2-en-8-oxo-2-carboxylic acid is suspended in 60 ml of nitromethane, and 1.05 ml of 1,8-diazabicyclo [5.4.0] undecene-7 is added thereto. The mixture is stirred at room temperature for 23 hours. The reaction mixture is poured into 200 ml of an ice cooled aqueous 0.5 N hydrochloric acid solution, and the mixture is extracted with 200 ml of ethyl acetate. The extract is washed successively three times with an aqueous saturated sodium chloride solution, twice with an aqueous saturated sodium hydrogen carbonate solution, twice with water and once with an aqueous saturated

sodium chloride solution, and then dried over anhydrous sodium sulfate. The solvent is removed therefrom by distillation. Then, the residue is crystallized from ethyl acetate-n-hexane to obtain 4.55 g of the desired compound (65%) having the following physical properties.

IR $\nu_{max}^{KBr}$ ($cm^{-1}$): 1790(sh), 1780(sh), 1770, 1730, 1560, 1385

NMR($CDCl_3$) $\delta$: 7.63 - 7.97(4H, m), 5.48(1H, d, J = 5.0Hz), 5.03(1H, dd, J = 10.5, 7.5Hz), 4.85(1H, dd, J = 10.5, 7.5Hz), 4.51(1H, br. s), 3.93 - 4.23 (1H, m), 2.87 - 3.17(1H, m), 1.43 - 1.93(4H, m), 1.50(9H, s)

<u>Example 2</u>

Tertiary butyl ester of (±)-6,7-cis-7β-phthalimido-1-azabicyclo [4.2.0] oct-3-nitromethyl-4α-hydroxy-8-oxo-2-carboxylic acid:

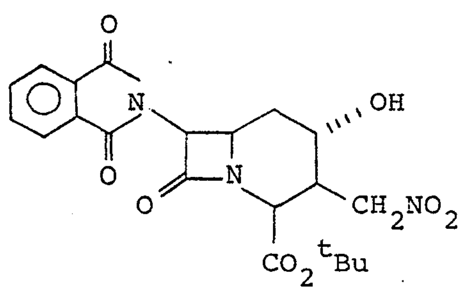

With the same treatment as in Example 1, using 7.4 g (19.2 m moles) of tertiary butyl ester of (±)-cis-7-β phthalimido-1-azabicyclo [4.2.0] oct-2-en-4α-hydroxy-8-oxo-2-carboxylic acid, 77 ml of nitromethane and 1.35 ml of 1,8-diazabicyclo [5.4.0] undecene-7, 5.54 g of the desired compound (64.6%) is obtained. The compound has the following physical properties.

IR $\nu_{max}^{KBr}$ ($cm^{-1}$): 3540, 1790(sh), 1780(sh), 1765, 1735, 1720(sh), 1710(sh), 1560, 1390

NMR($CDCl_3$-$CD_3OD$) $\delta$: 7.70 - 7.97(4H, m), 5.54(1H, d, J = 5.0Hz), 4.99(1H, dd, J = 13.5, 9.0Hz), 4.67

(1H, dd, J = 13.5, 6.0Hz), 4.50(1H, br. s), 3.93 - 4.43(2H, m), 3.16 - 3.47(1H, m), 1.47 - 2.03 (2H, m), 1.50(9H, s)

Example 3

Tertiary butyl ester of (±)-6,7-cis-7β-phthalimido-1-azabicyclo [4.2.0] oct-3-formyl-4α-hydroxy-8-oxo-2-carboxylic acid:

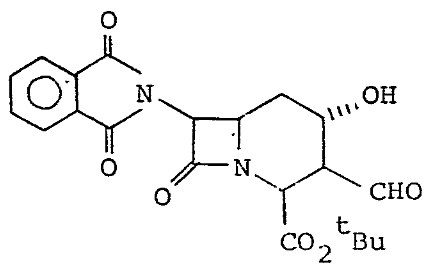

At first, 5.54 g (12.4 m moles) of tertiary butyl ester of (±)-6,7-cis-7β-phthalimido-1-azabicyclo [4.2.0] oct-3-nitromethyl-4α-hydroxy-8-oxo-2-carboxylic acid obtained in Example 2 is treated in the same manner as in Example 8-1) to obtain 2.66 g of crude desired product (51.6%). The crude product can be used in the successive step without any purification. A portion of the crude product is purified by silica gel chromatography (n-hexane - ethyl acetate = 1 : 3) to obtain the purified desired compound having the following physical properties. The compound is mainly a mixture of two isomers.

IR $\nu_{max}^{CHCl_3}$ ($cm^{-1}$): 1790, 1775(sh), 1770, 1730, 1720(sh)

NMR($CDCl_3$-$CD_3OD$)δ: 9.90(1H, s), 7.72 - 7.96(4H, m), 5.44 - 5.60(1H, m), 4.92 - 5.08(1H, m), 4.20 - 4.84 (2H, m), 2.36 - 2.60(1H, m), 1.56 - 2.20(2H, m), 1.52(9H, br. s)

Example 4

Tertiary butyl ester of (±)-6,7-trans-7-azido-1-azabicyclo [4.2.0] oct-3-formyl-8-oxo-2-carboxylic acid:

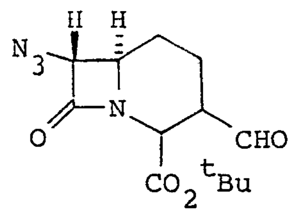

At first, 200 mg (0.62 m moles) of tertiary butyl ester of (±)-6,7-trans-7-azido-1-azabicyclo [4.2.0] oct-3-nitromethyl-8-oxo-2-carboxylic acid is dissolved in 6 ml of methanol, and a solution consisting of 1.2 g of ammonium acetate, 1.5 g of an aqueous 25% titanium trichloride solution and 6 ml of water is added thereto in 5 portions over 30 minutes. The mixture is stirred at room temperature for one hour. Then, 30 ml of water and 30 ml of ethyl acetate are added to the reaction mixture to conduct extraction. The extract is washed twice with an aqueous saturated sodium chloride solution and dried over anhydrous sodium sulfate, and the solvent is removed therefrom by distillation. The residue is purified by 40 g of silica gel, and fractions containing the desired compound, eluted by n-hexane - ethyl acetate are collected and concentrated to obtain 79 mg of an oily product (43.4%). The oily product has the following physical properties:

IR $\nu_{max}^{CHCl_3}$ ($cm^{-1}$): 2130, 1790(sh), 1780, 1750(sh), 1745, 1735(sh)

NMR($CDCl_3$)δ: 9.67(1H, s), 4.96(1H, br. s), 4.11(1H, d, J = 2.0Hz), 3.50 - 3.72(1H, m), 2.85 - 3.05(1H, m), 1.50(9H, s)

Example 5

Methyl ester of (6R,7S)-7-[2-(2-tritylamino-4-thiazolyl)-2-syn-methoxyiminoacetamido]-1-azabicyclo [4.2.0] oct-3-nitromethyl-8-oxo-2-carboxylic acid:

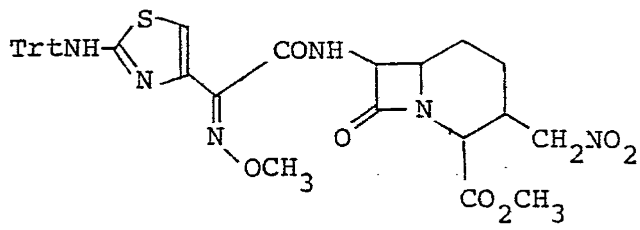

With the same treatment as in Example 1, using 610 mg (1 m mole) of methyl ester of (6R,7S)-7-[2-(2-tritylamino-4-thiazolyl)-2-syn-methoxyimino)-acetamido]-1-azabicyclo [4.2.0] oct-2-en-8-oxo-2-carboxylic acid, 64 μl of 1,8-diazabicyclo [5.4.0] undecene-7, 3.7 ml of nitromethane and 1 ml of chloroform, 540 mg of a crude product is obtained. The crude product is purified by 30 g of silica gel, and fractions containing the desired compound eluted by n-hexane - ethyl acetate ( = 1 : 1) are collected and concentrated under reduced pressure to obtain 380 mg of a solid (58.0%). The solid has the following phsyical properties:

IR $\nu_{max}^{KBr}$ ($cm^{-1}$): 1780(sh), 1775(sh), 1770(sh), 1760(sh), 1755, 1745(sh), 1695(sh), 1685, 1560.

NMR($CDCl_3$-$CD_3OD$) δ: 7.32(15H, s), 6.67(1H, s), 5.17 (1H, d, J = 5.0Hz), 4.43 - 4.80(3H, m), 3.98(3H, s), 3.80(3H, s), 2.87 - 3.20(1H, m), 1.43 - 2.00 (4H, m)

Example 6

Tertiary butyl ester of (±)-6,7-cis-7-azido-1-azabicyclo [4.2.0] oct-3-nitromethyl-8-oxo-2-carboxylic acid:

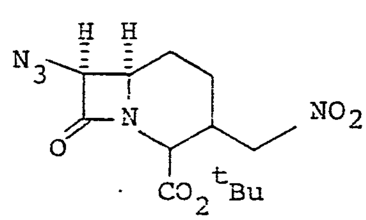

At first, 300 mg (1.14 m moles) of tertiary butyl ester of (±)-6,7-cis-7-azido-1-azabicyclo [4.2.0] oct-2-en-8-oxo-2-carboxylic acid is dissolved in 6 ml of nitromethane, and 100 µl of 1,8-diazabicyclo [5.4.0] undecene-7 is added thereto. The mixture is stirred at room temperature for 18 hours. Thereafter, the same treatment as in Example 1 and then purification with silica gel (30 g) are carried out. Fractions containing the desired compound, eluted by n-hexane - ethyl acetate (= 2 : 1) are collected and concentrated under reduced pressure to obtain 180 mg of an oily product (48.7%). The oily product has the following physical properties:

IR $\nu_{max}^{CDCl_3}$ ($cm^{-1}$): 2140, 1780, 1775(sh), 1755(sh), 1750, 1570

NMR($CDCl_3$) δ: 4.86(1H, d, J = 4.8Hz), 4.57(1H, dd, J = 12.0, 8.0Hz), 4.49(1H, dd, J = 12.0, 8.0Hz), 4.33(1H, d, J = 1.2Hz), 3.75 - 4.12(1H, m), 2.80 - 3.20(1H, m), 1.53(9H, s)

Example 7

Tertiary butyl ester of (±)-6,7-trans-7-azido-1-azabicyclo [4.2.0] oct-3-nitromethyl-8-oxo-2-carboxylic acid:

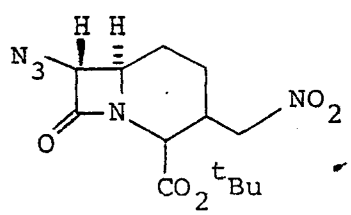

The same treatment as in Example 6 using 150 mg (0.57 m moles) of tertiary butyl ester of (±)-6,7-trans-7-azido-1-azabicyclo [4.2.0] oct-2-en-8-oxo-2-carboxylic acid and then chromatographic purification are carried out, whereby 70 mg of the desired compound is obtained as an oily product (37.9%). The product has the following physical properties:

IR $\nu_{max}^{CHCl_3}$ ($cm^{-1}$): 2130, 1785(sh), 1780, 1750, 1570

NMR($CDCl_3$) δ: 4.58(1H, dd, J = 12.0, 8.0Hz), 4.54 (1H, dd, J = 12.0, 8.0Hz), 4.24 - 4.36(2H, m), 3.60 - 3.84(1H, m), 2.84 - 3.16(1H, m), 1.51(9H, s)

Example 8

8-1) Tertiary butyl ester of (±)-6,7-cis-7-phthalimido-1-azabicyclo [4.2.0] oct-3-formyl-8-oxo-2-carboxylic acid:

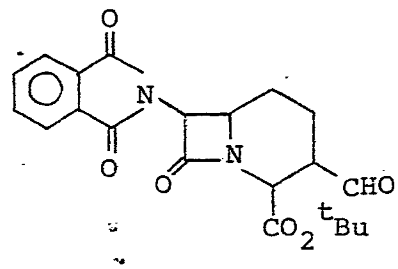

At first, 2.75 g (6.4 m moles) of tertiary butyl ester of (±)-6,7-cis-7-phthalimido-1-azabicyclo [4.2.0] oct-3-nitromethyl-8-oxo-2-carboxylic acid obtained in Example 1 is dissolved in 60 ml of 1,2-dimethoxyethane and 40 ml of methanol, and 70 ml of a solution consisting of 11.5 g of ammonium acetate, 15.5 g of an aqueous 25% titanium trichloride solution and water is added thereto in 7 portions over one hour. Then, the mixture is stirred at room temperature for one hour. Then, 200 ml of water and 200 ml of ethyl acetate are added to the reaction mixture to conduct extraction. The extract is washed successively twice with an aqueous saturated sodium chloride solution, once with an aqueous sodium hydrogen carbonate solution, twice with water and once with an aqueous saturated sodium chloride solution, and then dried over anhydrous sodium sulfate. By removing the solvent therefrom by distillation, 1.92 g of crude desired compound is obtained (74.9%). The crude compound can be used in the successive step without purification.

A portion of the crude compound is purified by silica gel chromatography (n-hexane - ethyl acetate = 1 : 1).

The purified desired compound has the following physical properties:

< More polar compound >

IR $\nu_{max}^{KBr}$ ($cm^{-1}$): 1795, 1780(sh), 1775, 1740(sh), 1730

NMR($CDCl_3$)$\delta$: 9.90(1H, s), 7.67 - 7.93(4H, m), 5.43(1H, d, J = 4.5Hz), 5.17(1H, br. s), 3.90 - 4.20(1H, m), 2.77 - 2.93(1H, m), 1.47 - 2.57(4H, m), 1.50(9H, s)

< Less polar compound >

IR $\nu_{max}^{KBr}$ ($cm^{-1}$): 1795, 1780(sh), 1775, 1740(sh), 1730

NMR($CDCl_3$)$\delta$: 9.80(1H, s), 7.70 - 7.97(4H, m), 5.50 (1H, d, J = 4.5Hz), 5.20(1H, d, J = 5.5Hz), 3.93 - 4.27(1H, m), 2.93 - 3.13(1H, m), 1.23 - 2.43(4H, m), 1.47(9H, s)

8-2) Tertiary butyl ester of (±)-6,7-cis-7-phthalimido-1-azabicyclo [4.2.0] oct-2-en-3-formyl-8-oxo-2-carboxylic acid:

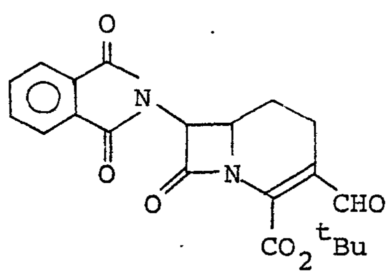

At first, 10.2 g of tertiary butyl ester of (±)-6,7-cis-7-phthalimido-1-azabicyclo [4.2.0] oct-3-formyl-8-oxo-2-carboxylic acid obtained in Example 8-1) is dissolved in 200 ml of anhydrous tetrahydrofuran, and 12.8 g of pyrrolidone hydrotribromide is added thereto. The mixture is stirred at 50°C for one hour. Then, the reaction mixture is cooled to 0°C, and 12.5 ml of triethylamine is added thereto with stirring, and stirring is further continued for 2 hours. The reaction mixture is poured into an aqueous 1 N hydrochloric acid solution under cooling,

and extracted with ethyl acetate. The organic layer is washed successively with water, an aqueous sodium sulfite solution, water and an aqueous saturated sodium chloride solution, and dried over anhydrous sodium sulfate. Then, the solvent is removed therefrom under reduced pressure, and the residue is crystallized from a mixed solvent system of ethyl acetate - dimethyl ether-n-hexane, whereby 2.36 g of the desired compound is obtained as a solid. Furthermore, the mother liquor is concentrated under reduced pressure, and the residue is purified by 100 g of silica gel (n-hexane - ethyl acetate = 1 : 1), whereby 1.26 g of the desired compound is further obtained (yield: 36%). The compound has the following physical properties:

NMR($CDCl_3$) $\delta$: 9.97(1H, s), 7.70 - 8.00(4H, m), 5.68 (1H, d, J = 5.0Hz), 3.90 - 4.20(1H, m), 2.77 - 3.10 (1H, m), 1.33 - 1.67(3H, m), 1.60(9H, s)

IR $\nu_{max}^{CHCl_3}$ ($cm^{-1}$): 1810(sh), 1800, 1790, 1730, 1725(sh), 1670, 1600

Example 9

Tertiary butyl ester of (±)-6,7-cis-7-phthalimido-1-azabicyclo [4.2.0] oct-2-en-3-formyl-8-oxo-2-carboxylic acid:

At first, 2.75 g (6.4 m moles) of tertiary butyl ester of (±)-6,7-cis-7-phthalimido-1-azabicyclo [4.2.0] oct-3-nitromethyl-8-oxo-2-carboxylic acid obtained in Example 1 is dissolved in 60 ml of 1,2-dimethoxyethane and 40 ml of methanol, and 70 ml of a solution consisting of 11.5 g of ammonium acetate, 15.5 g of an aqueous 25% titanium trichloride solution and water is added thereto in 7 portions over one hour. Then, the mixture is stirred at room temperature for one hour, and 200 ml of water and 200 ml of ethyl acetate are added thereto to conduct extraction. The extract is washed successively twice with an aqueous saturated sodium chloride solution, once with an aqueous sodium hydrogen carbonate solution, twice with water, and once with a saturated aqueous sodium chloride solution,

and dried over anhydrous sodium sulfate. The solvent is removed therefrom by distillation, and 35 ml of anhydrous tetrahydrofuran is added to the residue. Then, 2.4 g of pyrrolidone hydrotribromide is added thereto. The mixture is stirred at 50°C for one hour. Then, the reaction mixture is cooled to 0°C, and 2.4 ml of triethylamine is added thereto with stirring. The mixture is stirred as such for two hours. The reaction mixture is poured into an aqueous 1 N hydrochloric acid with cooling, and extracted with ethyl acetate. The organic layer is washed successively with water, an aqueous sodium sulfite solution and an aqueous saturated sodium chloride solution, and dried over anhydrous sodium sulfate. The solvent is removed therefrom by distillation. The residue is crystallized from a mixed solvent system of ethyl acetate - diethyl ether-n-hexane, whereby 631 mg of the desired compound is obtained as a solid. The mother liquor is purified by 20 g of silica gel (n-hexane : ethyl acetate = 1 : 1), whereby 303 mg of the desired compound is further obtained (yield: 35%).

The physical properties of the desired compound are quite identical with those obtained in Example 8-2).

Reference Example 1

Tertiary butyl ester of (±)-cis-7-phthalimido-1 azabicyclo [4.2.0] oct-2-en-3-hydroxy-8-oxo-2-carboxylic acid:

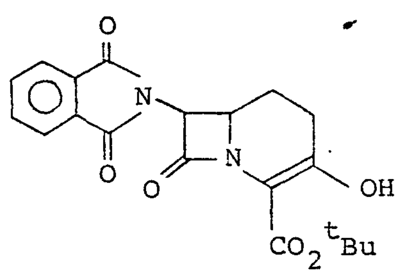

At first, 200 mg (0.5 m moles) of tertiary butyl ester of (±)-6,7-cis-7-phthalimido-1-azabicyclo [4.2.0] oct-3-formyl-8-oxo-2-carboxylic acid obtained in Example 8-1)

is dissolved in 300 μl of anhydrous dimethylformamide, and 168 μl (1.2 m moles) of triethylamine and 76 μl (0.6 m moles) of chlorotrimethylsilane are added thereto. The mixture is stirred at 90°C for 4 hours, and then cooled and poured into 10 ml of ice-cooled aqueous saturated sodium hydrogen carbonate solution with 20 ml of ethyl acetate. The extract is washed twice with water and once with an aqueous saturated sodium chloride solution, and dried over anhydrous sodium sulfate. By removing the solvent therefrom by distillation, 220 mg of silylenol ether is obtained as a crude oil. The crude oil is dissolved in 3 ml of ethyl acetate, and ozone is passed through the solution with ice cooling. After excess ozone is detected in the effluent gas (15 minutes), 1 ml of dimethyl sulfide is added to the reaction mixture, and the mixture is left standing at room temperature for 30 minutes. The solvent is removed therefrom by distillation, and the residue is purified by 10 g of silica gel, and fractions containing the desired compound, eluted by n-hexane - ethyl acetate (= 2 : 1) are collected, and concentrated under reduced pressure to obtain 36 mg of an oily product (18.8%). The oily product has the following physical properties:

IR $\nu_{max}^{CHCl_3}$ ($cm^{-1}$): 3020, 2990, 1795, 1775, 1740(sh), 1730, 1665, 1620

NMR($CDCl_3$)$\delta$: 11.54(1H, s), 7.70 - 7.97(4H, m), 5.54(1H, d, J = 5.0Hz), 3.73 - 4.07(1H, m), 1.7 - 3.2(4H, m), 1.58(9H, s)

Reference Example 2

Tertiary butyl ester of (±)-cis-7-phthalimido-1-azabicyclo [4.2.0] oct-2-en-3-methoxy-8-oxo-2-carboxylic acid:

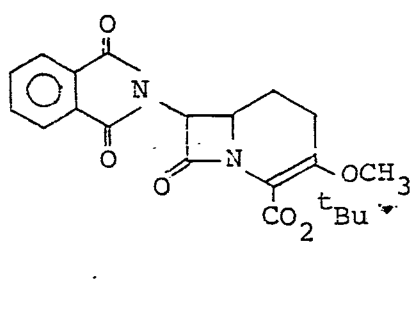

At first, 42.8 mg (0.111 m moles) of tertiary butyl ester of (±)-cis-7-phthalimido-1-azabicyclo [4.2.0] oct-2-en-3-hydroxy-8-oxo-2-carboxylic acid as obtained in Reference Example 1 is dissolved in 3 ml of chloroform and 1 ml of ethyl acetate, and 4 ml of an ether solution of diazomethane obtained according to the procedure disclosed in Organic Synthesis Vol.2, page 165, is added thereto. The mixture as such is stirred at room temperature for 3 hours. After addition of 100 μl of acetic acid thereto, the solvent is removed therefrom by distillation, and the residue is purified by 10 g of silica gel. Fractions containing the desired compound, eluted by n-hexane - ethyl acetate (= 1 : 1) are collected and concentrated under reduced pressure to obtain 22.2 mg of the desired oily product (50.0%). The oily product has the following physical properties:

IR $\nu_{max}^{CHCl_3}$ ($cm^{-1}$): 1790(sh), 1780(sh), 1770, 1730

NMR($CDCl_3$)δ: 7.67 - 7.97(4H, m), 5.53(1H, d, J = 5.5Hz), 3.67 - 3.97(1H, m), 3.73(3H, s), 1.77 - 2.87(4H, m), 1.53(9H, s)

Reference Example 3

Tertiary butyl ester of (±)-6,7-cis-7-phthalimido-1-azabicyclo [4.2.0] oct-3-en-3-formyl-8-oxo-2-carboxylic acid:

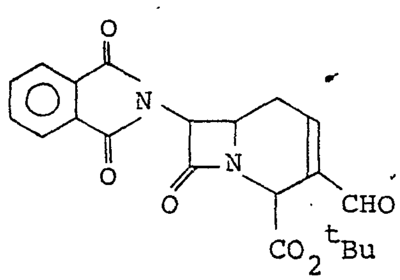

At first, 2.66 g (6.4 m moles) of tertiary butyl ester of (±)-6,7-cis-7β-phthalimido-1-azabicyclo [4.2.0] oct-3-formyl-4α-hydroxy-8-oxo-2-carboxylic acid obtained in Example 3 is dissolved in 60 ml of anhydrous methylene

chloride, and 2.72 ml of triethylamine is added thereto under ice cooling, and then 1.01 ml of methanesulfonyl chloride is added thereto. The mixture as such is stirred for 45 minutes. The reaction mixture is poured into ice water, and the mixture is extracted with ethyl acetate. After water washing, the organic layer is dried over anhydrous sodium sulfate, and then the solvent is removed therefrom by distillation. The oily product is purified by 60 g of silica gel, and fractions containing the desired compound, eluted by n-hexane - ethyl acetate (= 2 : 3) are collected and concentrated under reduced pressure to obtain 665 mg of a solid (26.1%). The solid has the following physical properties:

IR $\nu_{max}^{CHCl_3}$ ($cm^{-1}$): 1795(sh), 1785(sh), 1780, 1740(sh), 1735, 1700

NMR($CDCl_3$)δ: 9.50(1H, s), 7.67 - 7.90(4H, m), 6.87 - 7.07(1H, m), 5.58(1H, d, J = 5.0Hz), 5.13 (1H, d, J = 1.5Hz), 4.03 - 4.33(1H, m), 2.43 - 2.70 (1H, m), 1.47(9H, s)

Reference Example 4

Tertiary butyl ester of (±)-6,7-cis-7-phthalimido-1-azabicyclo [4.2.0] oct-3-en-3-hydroxymethyl-8-oxo-2-carboxylic acid:

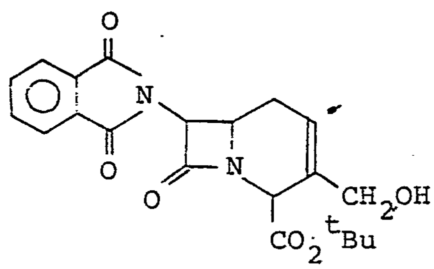

At first, 100 mg (0.25 m moles) of tertiary butyl ester of (±)-6,7-cis-7-phthalimido-1-azabicyclo [4.2.0] oct-3-en-3-formyl-8-oxo-2-carboxylic acid obtained in Reference Example 3 is dissolved in 8 ml of methanol, and 20 mg of bromocresol green is added thereto as an indicator. With

stirring at room temperature, 100 mg of cyanoboron sodium hydride is added thereto in 4 portions, while pH is adjusted with 1 N hydrochloric acid so that the color of the reaction mixture turns yellow. After the addition, stirring is continued for 30 minutes, and the reaction mixture is poured into an aqueous 1 N hydrochloric acid solution under ice cooling. The mixture is extracted with ethyl acetate. The organic layer is washed with water and dried over anhydrous sodium sulfate. The solvent is removed therefrom by distillation, and the residue is purified by 10 g of silica gel. Fractions containing the desired compound, eluted by n-hexane - ethyl acetate (= 1 : 2) are collected and concentrated under reduced pressure to obtain 78 mg of a solid. The solid has the following physical properties:

IR $\nu_{max}^{CHCl_3}$ ($cm^{-1}$): 1790(sh), 1780(sh), 1775, 1735, 1730(sh)

NMR($CDCl_3$-$CD_3OD$) $\delta$: 7.70 - 7.95(4H, m), 5.83 - 6.00(1H, m), 5.57(1H, d, J = 5.0Hz), 4.95(1H, br. s), 4.00 - 4.33(3H, m), 2.13 - 2.37(2H, m), 1.50(9H, s)

Reference Example 5

Tertiary butyl ester of (±)-6,7-cis-7-phthalimido-1-azabicyclo [4.2.0] oct-2-en-3-hydroxymethyl-8-oxo-2-carboxylic acid:

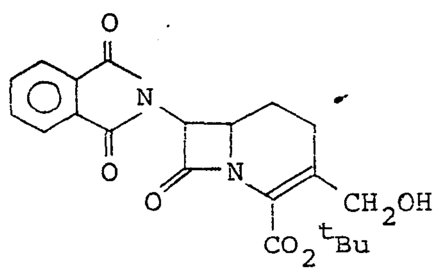

At first, 78 mg (0.196 m moles) of tertiary butyl ester of (±)-6,7-cis-7-phthalimido-1-azabicyclo [4.2.0] oct-3-en-3-hydroxymethyl-8-oxo-2-carboxylic acid obtained in Reference Example 4 is dissolved in 3 ml of anhydrous methylene chloride, and 100 µl of 1,8-diazabicyclo [5.4.0]

undecene-7 is added thereto. The mixture is stirred at room temperature for 20 minutes. The reaction mixture is poured into an ice-cooled aqueous 1 N hydrochloric acid solution, and the mixture is extracted with ethyl acetate. The organic layer is washed with water, and dried over anhydrous sodium sulfate. The solvent is removed therefrom by distillation, and the residue is purified by 10 g of silica gel. Fractions containing the desired compound, eluted by n-hexane - ethyl acetate (1 : 2) are collected and concentrated under reduced pressure to obtain 46 mg of a solid (59.0%). The solid has the following physical properties:

IR $\nu_{max}^{CHCl_3}$ ($cm^{-1}$): 1790, 1780(sh), 1775, 1730, 1725(sh)

NMR($CDCl_3$-$CD_3OD$)$\delta$: 7.73 - 8.00(4H, m), 5.60(1H, d, J = 5.5Hz), 4.43(1H, d, J = 12.0Hz), 4.20(1H, d, J = 12.0Hz), 3.70 - 4.10(1H, m), 1.73 - 2.63(4H, m), 1.55(9H, s)

Reference Example 6

Tertiary butyl ester of (±)-6,7-cis-7-phthalimido-1-azabicyclo [4.2.0] oct-2-en-3-[2-(1-methyl-1H-tetrazol-5-yl) vinyl]-8-oxo-2-carboxylic acid:

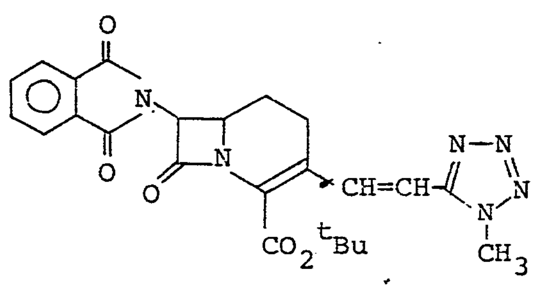

At first, 600 mg of tertiary butyl ester of (±)-6,7-cis-7-phthalimido-1-azabicyclo [4.2.0] oct-2-en-3-formyl-8-oxo-2-carboxylic acid obtained in Example 8-2) is dissolved in 20 ml of anhydrous tetrahydrofuran, and 1.1 g of (1-methyl-1H-tetrazol-5-yl) methylenetriphenylphosphorane is added thereto. The mixture is stirred at room temperature for 24 hours. The reaction mixture is concentrated

under reduced pressure, and the residue is dissolved in chloroform. The solution is purified by 100 g of silica gel (ethyl acetate : chloroform : n-hexane = 3 : 1 : 1) to obtain 520 mg of the desired compound as a solid (yield: 72%).

The desired compound has the following physical properties:

NMR($CDCl_3$)$\delta$: 8.17(1H, d, J = 16.5Hz), 7.70 - 7.93(4H, m), 6.51(1H, d, J = 16.5Hz), 5.65(1H, d, J = 6.0 Hz), 4.07(3H, s), 3.90 - 4.17(1H, m), 1.90 - 3.00 (4H, m), 1.60(9H, s)

IR $\nu_{max}^{KBr}$ ($cm^{-1}$): 1795, 1785, 1775(sh), 1730, 1725(sh), 1625

Reference Example 7

Tertiary butyl ester of (±)-6,7-cis-7-phthalimido-1-azabicyclo [4.2.0] oct-2-en-3-(2-ethoxycarbonylvinyl)-8-oxo-2-carboxylic acid:

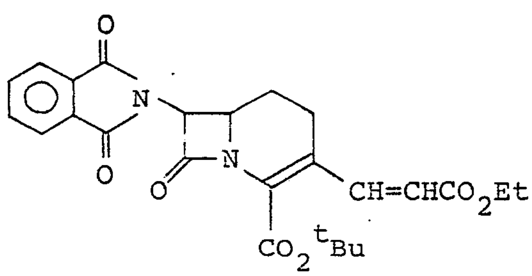

At first, 273 mg of tertiary butyl ester of (±)-6,7-cis-7-phthalimido-1-azabicyclo [4.2.0] oct-2-en-3-formyl-8-oxo-2-carboxylic acid obtained in Example 8-2) is dissolved in 14 ml of toluene, and 32 mg of (carbethoxymethylene) triphenylphosphorane is added thereto. The mixture is stirred at 60°C for 4 hours. The reaction mixture is concentrated under reduced pressure, and the residue is dissolved in chloroform. The solution is purified by 50 g of silica gel (n-hexane : ethyl acetate = 2 : 1) to obtain 190 mg of the desired compound as a solid (yield: 59%). The compound has the following physical properties:

NMR($CDCl_3$)δ: 8.03(1H, d, J = 16.5Hz), 7.67 - 7.90(4H, m), 5.95(1H, d, J = 16.5Hz), 5.63(1H, d, J = 6.0Hz), 4.20(2H, q, J = 7.5Hz), 3.83 - 4.10(1H, m), 1.77 - 2.90(4H, m), 1.60(9H, s), 1.27(3H, t, J = 7.5Hz)

IR $\nu_{max}^{CHCl_3}$ ($cm^{-1}$): 1800(sh), 1795, 1785(sh), 1780, 1730, 1725(sh), 1710(sh), 1620

Reference Example 8

Tertiary butyl ester of (±)-6,7-cis-7-phthalimido-1-azabicyclo [4.2.0] oct-2-en-3-(2-cyanovinyl)-8-oxo-2-carboxylic acid:

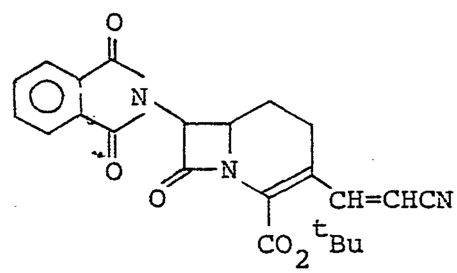

At first, 1 g of tertiary butyl ester of (±)-6,7-cis-7-phthalimido-1-azabicyclo [4.2.0] oct-2-en-3-formyl-8-oxo-2-carboxylic acid obtained in Example 8-2) is dissolved in 28 ml of anhydrous tetrahydrofuran, and 1.1 g of cyanomethylenetriphenylphosphorane is added thereto. The mixture is stirred at room temperature for 18 hours. The reaction mixture is concentrated under reduced pressure, and the residue is dissolved in chloroform. The solution is purified by 120 g of silica gel (n-hexane : ethyl acetate = 1 : 1) to obtain 708 mg of the desired compound as a solid (yield: 67%).

The compound has the following physical properties:

NMR($CDCl_3$)δ: 7.70 - 7.93(4H, m), 7.50(1H, d, J=13.5Hz), 5.67(1H, d, J = 5.5Hz), 5.30(1H, d, J = 13.5Hz), 3.90 - 4.13(1H, m), 3.07 - 3.43(1H, m), 2.37 - 2.90 (1H, m), 1.77 - 2.36(2H, m), 1.57(9H, s)

IR $\nu_{max}^{KBr}$ ($cm^{-1}$): 2225, 1805(sh), 1800, 1790, 1780(sh), 1735(sh), 1730

Reference Example 9

(±)-6,7-cis-7-[2-(2-aminothiazol-4-yl)-2-syn-methoxyiminoacetamido]-3-[2-(1-methyl-1H-tetrazol-5-yl) vinyl]-1-azabicyclo [4.2.0] oct-2-en-8-oxo-2-carboxylic acid:

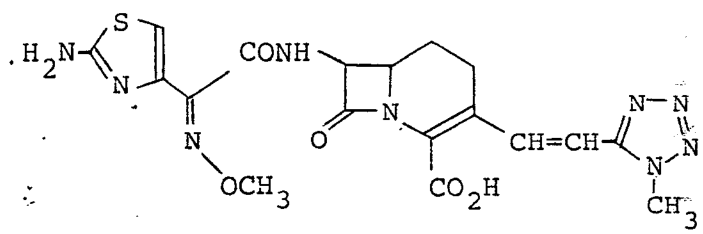

At first, 540 mg of tertiary butyl ester of (±)-6,7-cis-7-phthalimido-1-azabicyclo [4.2.0] oct-2-en-3-[2-(1-methyl-1H-tetrazoly-5-yl) vinyl]-8-oxo-2-carboxylic acid obtained in Reference Example 6 is dissolved in 15.6 ml of tetrahydrofuran and 2.7 ml of water, and a solution of 375 mg of sodium sulfide nonahydrate in 3.52 ml of water is added thereto under ice cooling. The mixture is stirred for 15 minutes. The reaction mixture is adjusted to pH 1.5 with 1 N hydrochloric acid, and extracted twice with ethyl acetate. The organic layer is washed with an aqueous saturated sodium chloride solution, and dried over anhydrous sodium sulfate. The solvent is removed therefrom under reduced pressure, and 6.8 ml of anhydrous tetrahydrofuran is added to the residue. Then, 281 mg of N,N'-dicyclohexylcarbodiimide is added thereto. The mixture is stirred at room temperature for 2 hours. The resulting precipitate is removed therefrom by filtration, and the filtrate is cooled to -78°C. Then, 72 µl of methylhydrazine is added to the filtrate, and the mixture is stirred as such for 20 minutes. Then, 1.2 ml of 1 N hydrochloric acid is added thereto, and the temperature of the mixture is returned to room temperature over 20 minutes.

The mixture is further stirred as such for 30 minutes. Then, 50 ml of water and 2 ml of 1 N hydrochloric acid are added to the reaction mixture. The water layer is washed three times with ethyl acetate, adjusted to pH 8 with an aqueous saturated sodium hydrogen carbonate solution under cooling and then extracted three times with ethyl acetate. The organic layer is washed with an aqueous saturated sodium chloride solution, and dried over anhydrous sodium sulfate. Then, 0.8 ml of trifluoroacetic acid is added to the organic layer, and the solvent is removed therefrom under reduced pressure. Then, 7 ml of anhydrous methylene chloride is added to the residue, and then 7 ml of trifluoroacetic acid is added thereto. The mixture is left standing at room temperature for one hour. The solvent is removed therefrom under reduced pressure. Then, 5 ml of ethyl acetate is added to the residue, and the solvent is removed again therefrom under reduced pressure. The residue is pulverized in ether, whereby 135 mg of a solid is obtained. The solid is dissolved in a solution consisting of 3.7 ml of water and 2.2 ml of tetrahydrofuran, and the solution is adjusted to pH 7.7 with triethylamine.

On the other hand, 266 mg of 2-(2-tritylamino-4-thiazolyl)-2-syn-methoxyiminoacetic acid is dissolved in 3 ml of anhydrous tetrahydrofuran, and 84 µl of triethylamine is added thereto under ice cooling. Then, 125 mg of phosphorus pentachloride is added thereto, and the mixture is stirred at the same temperature for one hour to obtain an acid chloride solution. The acid chloride solution is added dropwise to the above-mentioned solution, while keeping the mixture to pH 7.5 - 8.0 with triethylamine. Then, the mixture is stirred at the same temperature for one hour. The mixture is adjusted to pH 1.5 with 1 N hydrochloric acid, an aqueous saturated sodium chloride solution is added thereto, and the mixture is extracted twice with ethyl acetate. The organic layer is washed with an aqueous saturated sodium chloride solution, and the solvent is removed therefrom under reduced pressure. Then, 20 ml of an aqueous 50% acetic acid solution is added to

the residue, and the mixture is stirred at 50°C for one hour. The solvent is removed therefrom by distillation under reduced pressure, and the residue is dissolved in a small amount of dimethylsulfoxide. The solution is passed through a column of Diaion HP 10 (30 ml), and the column is washed with water. Fractions containing the desired compound, eluted by water - methanol (= 3 : 1) are collected, and the solvent is removed therefrom by distillation under reduced pressure to obtain 36 mg of the desired compound as a solid (yield: 7%).

The compound has the following physical properties:

NMR($D_2O$-$CD_3OD$-$d_6DMSO$) δ: 8.10(1H, d, J = 16.5Hz), 6.92(1H, s), 6.73(1H, d, J = 16.5Hz), 5.52(1H, d, J = 5.5Hz), 4.10(3H, s), 3.90 - 4.17(1H, m), 4.00 (3H, s), 1.67 - 3.10(4H, m)

IR $\nu_{max}^{KBr}$ ($cm^{-1}$): 1780(sh), 1770, 1755(sh), 1660, 1620

Reference Example 10

(±)-6,7-cis-7-[2-(2-aminothiazol-4-yl)-2-syn-methoxyiminoacetamido]-3-(2-ethoxycarbonylvinyl)-1-azabicyclo [4.2.0]oct-2-en-8-oxo-2-carboxylic acid:

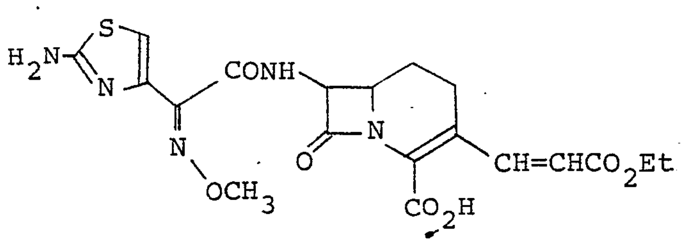

Deprotection and acylation reaction are carried out in the same manner as in Reference Example 9 with 390 mg of tertiary butyl ester of (±)-6,7-cis-7-phthalimido-1-azabicyclo [4.2.0] oct-2-en-3-(2-ethoxycarbonylvinyl)-8-oxo-2-carboxylic acid obtained in Reference Example 7 to obtain 40 mg of the desired compound as a solid (yield: 14%). The compound has the following physical properties:

NMR($D_2O$-$CD_3OD$-$d_6DMSO$) δ: 7.95(1H, d, J = 16.4Hz), 6.81(1H, s), 6.10(1H, d, J = 16.4Hz), 5.53(1H, d, J = 5.2Hz), 4.19(2H, q, J = 7.0Hz), 3.94(3H, s), 1.27(3H, t, J = 7.0Hz)

IR $\nu_{max}^{KBr}$ ($cm^{-1}$): 1790(sh), 1780, 1770(sh), 1760(sh), 1670, 1630

Reference Example 11

Tertiary butyl ester of (±)-6,7-cis-7-phthalimido-1-azabicyclo [4.2.0] oct-2-en-3-cyano-8-oxo-2-carboxylic acid:

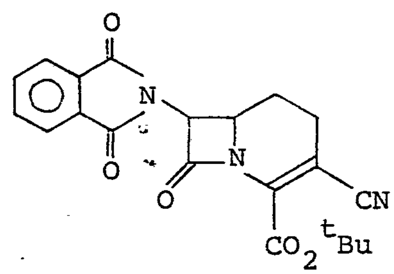

At first, 800 mg of tertiary butyl ester of (±)-6,7-cis-7-phthalimido-1-azabicyclo [4.2.0] oct-2-en-3-formyl-8-oxo-2-carboxylic acid obtained in Example 8-2) is suspended in 10 ml of toluene. Then, 550 µl of pyridine and 960 mg of O,N-bis-(trifluoroacetyl)-hydroxyamine are added thereto, and the mixture is stirred with heating at 85°C for one hour. The reaction mixture is cooled, water and ethyl acetate are added thereto, and the organic layer is separated. The organic layer is washed successively with water, an aqueous sodium hydrogen carbonate solution and an aqueous saturated sodium chloride solution, and the organic layer is dried over anhydrous sodium sulfate. The organic layer is subjected to distillation under reduced pressure, and the residue is crystallized from ethyl acetate - chloroform - n-hexane to obtain 596 mg of the desired cyano compound. The mother liquor is purified with 70 g of silica gel (chloroform - n-hexane - ethyl acetate = 1 : 1 : 1) to obtain 124 mg of the desired compound (yield:

91.5%).

The compound has the following physical properties:

NMR($CDCl_3$)δ: 7.7 - 8.0(4H, m), 5.70(1H, d, J = 5.5Hz), 3.83 - 4.23(1H, m), 2.37 - 2.73(2H, m), 1.80 - 2.17 (2H, m), 1.59(9H, s)

IR $\nu_{max}^{KBr}$ ($cm^{-1}$): 2230, 1810(sh), 1805, 1795(sh), 1780(sh), 1740(sh), 1735, 1725(sh)

Reference Example 12

Antibacterial effects of the final useful compounds by dilution method on Mueller-Hinton agar (pH 7.0) are shown in the following table (MIC μg/mℓ).

| Microorganism * | 1 | 2 | 3 | 4 | 5 | 6 | 7 | 8 |
|---|---|---|---|---|---|---|---|---|
| Compound of Ref. Ex. 9 | 1.56 | 0.01 | 0.1 | 1.56 | 0.01 | 0.01 | 0.2 | 0.2 |
| Compound of Ref. Ex. 10 | 6.25 | 0.2 | 0.39 | 3.13 | 0.1 | 0.01 | 1.56 | 0.78 |

* 1 Staphylococcus aureus 209-P
2 Escherichia coli NIHJJC-2
3 Krebsiella pneumoniae 8045
4 Serratia marcescens T-26
5 Proteus mirabilis 1287
6 Proteus vulgaris 6897
7 Enterobacter cloacae F 1510
8 Citrobacter freundii F 1526

WHAT IS CLAIMED IS:

1. A β-lactam compound represented by the general formula [I]:

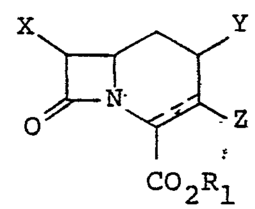

[ I ]

[wherein X is phthalimido, azido, phenylacetamido, phenoxyacetamido, 2-thienylacetamido, or

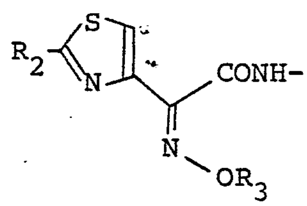

(wherein $R_2$ is amino or protected amino, $R_3$ is hydrogen or methyl); Y is hydrogen, methyl or hydroxyl; Z is $-CHR_4NO_2$ (wherein $R_4$ is hydrogen or straight or branched alkyl of $C_1-C_4$) or formyl; $R_1$ is straight or branched alkyl of $C_1-C_5$ (which can be substituted by halogen), substituted silyl, or benzyl, benzhydryl or trityl (whose phenyl nuclei can be substituted by methyl, methoxy or nitro); ----- represents single bond when Z is $-CHR_4NO_2$ and represents single or double bond when Z is formyl; and in spite of the above definition, when X is 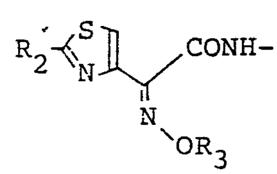 , Z is $-CHR_4NO_2$].

2. A β-lactam compound according to claim 1, wherein the protecting group of the protected amino for $R_2$ is trityl, t-butyloxycarbonyl, 2,2,2-trichloroethyloxycarbonyl, chloroacetyl or formyl.

3. A β-lactam compound according to claim 1, wherein the substituted silyl under $R_1$ is methylsilyl or t-butyldimethylsilyl.

4. A β-lactam compound according to claim 1, wherein X is phthalimido, Y is hydrogen, Z is formyl and ----- represents single or double bond.

5. A β-lactam compound according to claim 1, wherein Z is $-CHR_4NO_2$ (wherein $R_4$ has the same meaning as defined above) and ----- represents single bond.

0112481

European Patent Office

# EUROPEAN SEARCH REPORT

Application number

EP 83 11 1375

| DOCUMENTS CONSIDERED TO BE RELEVANT | | | |
| --- | --- | --- | --- |
| Category | Citation of document with indication, where appropriate, of relevant passages | Relevant to claim | CLASSIFICATION OF THE APPLICATION (Int. Cl. 3) |
| A | EP-A-0 014 476 (KYOWA)<br>* Claim 1 *<br>--- | 1 | C 07 D 471/04<br>A 61 K 31/435//<br>(C 07 D 471/04<br>C 07 D 221/00<br>C 07 D 205/00 ) |
| A | EP-A-0 025 602 (KYOWA)<br>* Claim 1 *<br>----- | 1 | |
| | | | TECHNICAL FIELDS SEARCHED (Int. Cl. 3) |
| | | | C 07 D 471/00<br>A 61 K 31/00 |

The present search report has been drawn up for all claims

| Place of search | Date of completion of the search | Examiner |
| --- | --- | --- |
| THE HAGUE | 08-02-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document